# EUROPEAN PATENT SPECIFICATION

(11) **EP 4 262 668 B1**
(45) Date of publication and mention of the grant of the patent: **12.02.2025**
(21) Application number: 21836167.3
(22) Date of filing: 14.12.2021
(51) Int. Cl.: A61H 1/00, A61H 7/00, A63B 24/00, A63B 26/00

(54) **SYSTEM FOR AT LEAST SUPPORTING A TREATMENT OF A PERSON AND USE OF SUCH A SYSTEM**
SYSTEM ZUR ZUMINDEST UNTERSTÜTZENDEN BEHANDLUNG EINER PERSON UND VERWENDUNG EINES SOLCHEN SYSTEMS
SYSTÈME POUR AU MOINS SUPPORTER UN TRAITEMENT D'UNE PERSONNE ET UTILISER UN TEL SYSTÈME

(30) Priority: 15.12.2020 NL 2027107
(43) Date of publication of application: 25.10.2023
(73) Proprietor: REFLEX SCIENCES B.V., 4462 RA Goes (NL)
(72) Inventor: PIEKE, Justus Johannes, 4462 RA Goes (NL)
(74) Representative: Custos IPC Coöperatie U.A.
(86) International application number: PCT/EP2021/085660
(87) International publication number: WO 2022/129030

(56) References cited:
- EP-B1- 2 811 963
- JP-A- 2000 060 934
- US-A1- 2002 068 884

## Description

The invention relates to a system for at least supporting a treatment of a person, comprising a base platform for receiving said person.

Document EP2811963B1 discloses a system according to the preamble of claim 1.

Said system may for example be used in or for a reflex treatment.

Said system may be used only as a supportive system in said treatment, in particular by bringing said person in a balanced position, but may also be used for the treatment itself. The system may thus alternatively be referred to as a supportive system for a treatment and/or a treatment system.

In accordance with the invention said base platform comprises at least one upper surface which defines a substantially flat reference plane, and at least one fill element, wherein said at least one fill element is displaceable between a first position and a second position, in which second position an upper surface of said at least one fill element extends above said reference plane with an adjustable distance, such that said at least one fill element fills a space between said reference plane and a lower side of a or each foot of said person.

In use of said system said person will stand on said upper surface of said base platform. The upper surface forms a substantially flat receiving surface to stand on, which defines said flat reference plane. Said at least one fill element is used for filling the space between the lower side of the or each foot of the person and said reference plane, i.e. the space defined by the foot arch. By filling this space said person is able to stand in a balanced position, which may support said treatment. Because every foot and thereby every foot arch is different, said at least one fill element is displaceable, such that said at least one fill element can be displaced in accordance with the or each foot of the person. It is noted that said at least one fill element may be part of the upper surface of the base platform. At least in the second position of the at least one fill element said upper surface may thus be substantially flat except in the area of the at least one fill element. Said flat reference plane is defined along the substantially flat surface part of the upper surface, excluding the area where said at least one fill element is arranged.

It is noted that any number of fill elements may be provided. For example one fill element may be provided that fills up the spaces below each foot. Practically at least two fill elements are provided, one for each foot, such that the adjustable distance of each fill element in the second positions can be adjusted independent of the other fill element. If desired even more fill elements may be provided, for example three, four, five, six, seven, or eight fill elements, preferably equally divided per foot.

It is noted that the first position of said at least one fill element may be chosen as desired. For example said at least one fill element may substantially not extend above said reference plane in said first position, such that said upper surface may be substantially completely flat in the first position of the at least one fill element. Alternatively said at least one fill element may extend slightly above said reference plane in the first position, but preferably not as high as in the second position thereof. In other words, the distance between the reference plane and an upper area of said at least one fill element is smaller in the first position of the at least one fill element than in the second position thereof.

It is noted that said at least one fill element is in particular displaceable while being fixedly attached to the base platform.

It is further noted that at least the upper surface of the fill element is displaceable.

Said system may optionally comprising a controller for controlling the adjustment of the at least one fill element.

In an embodiment of the system according to the invention said system comprises measuring means for measuring a pressure of the or each foot of the person, wherein said system is arranged to adjust the adjustable distance of said at least one fill element based on the measured pressure and/or comprises displaying means for displaying the measured pressure.

By measuring said pressure of the or each foot of the person and adjusting the at least one fill element in accordance therewith it can be achieved that said person stands on said base platform in a balanced position.

By means of displaying the measured pressure a person may adjust the adjustable distance of the at least one fill element in accordance with the displayed pressure. This person may be the person standing on the platform but also another person, for example a practitioner. Alternatively adjusting the adjustable distance of the at least one fill element can be performed automatically by said system being arranged to adjust the at least one fill element based on the measured pressure. Said controller may communicate with said measuring means and be arranged to control the adjustment of the at least one fill element based on said measured pressure.

Said pressure may be displayed in any suitable way, for example on a display screen or in a auditive manner.

Said measuring means may for example comprise at least one pressure sensor or any other suitable means for measuring a pressure.

In an embodiment of the system according to the invention said adjustable distance is adjusted such that:
- a pressure on both feet is substantially equal; and/or
- a pressure on a front part of the foot is equal to a pressure on a rear part of the foot; and/or
- a pressure on a left part of the foot is equal to a pressure on a right part of the foot.

In particular said system may be arranged to adjust the adjustable distance of the at least one fill element in such a manner, for example based on said measured pressure, but alternatively a said person may adjust the adjustable distance of the at least one fill element in such a manner, for example based on said measured and displayed pressure.

By obtaining any of the above described pressure conditions a balanced position of the person standing on the base platform may be obtained.

In an embodiment of the system according to the invention said system comprises a driving means for displacing the at least one fill element between its first and second position, wherein said driving means are arranged for displacing the at least one fill element mechanically and/or by means of a gas and/or by means of a liquid.

In a mechanical embodiment said at least one fill element could be displaced using any suitable mechanical driving means, wherein said mechanical driving means are for example powered by a motor. When a gas or liquid is used said at least one fill element could be a hollow element that can be filled with said gas or liquid and thereby expands and displaces from its first position to its second position, and said gas or liquid can be discharged such that the hollow element can shrink and displace back towards its first position.

Said driving means may optionally be part of the base platform.

In an embodiment of the system according to the invention said upper surface of the at least one fill element defines a number of surface area zones, wherein the adjustable distance is variable and/or individually adjustable per surface area zone.

Such an embodiment may provide the advantage that the height adjustment can be more accurate.

In an embodiment of the system according to the invention said at least one fill element has a shape that is substantially adapted to the or each foot of said person.

More in particular said shape may be substantially adapted to the foot arch.

As a result of such a shape the height and thereby the adjustable distance of the upper surface is substantially continuously variable over substantially the complete upper surface of the at least one fill element.

In an embodiment of the system according to the invention said at least one fill element is provided as a replaceable element.

An advantage thereof is that the fill element may be replaceable for a fill element having a shape that is adapted to the foot arch of a specific user.

In an embodiment of the system according to the invention said displacement takes place using rotation and/or translation of the at least one fill element.

By rotating and/or translating the at least one fill element, wherein said rotation and/or translation may be different for different areas of the at least one fill element, said adjustable distance can be easily and/or accurately adjustable.

Said rotation and/or translation of the at least one fill element may in particular be with respect to and/or out of the flat reference plane when said at least one fill element is displaced from the first position into the second position, and with respect to and/or into the flat reference plane if it is displaced back into the first position.

In an embodiment of the system according to the invention said at least one fill element is arranged and/or a distance between at least two fill elements is chosen such that a straight line is defined between an outer side of a hip bone and a second toe of the person when said person stands on said base platform and said at least one or two fill element(s) fill(s) said space.

In such an embodiment said person may be in a substantially stable position.

In an embodiment of the system according to the invention said system comprises at least one treatment element that extends out of said reference plane.

Such at least one treatment element can be used in the treatment of said person.

Such at least one treatment element projects upwards with respect to said reference plane.

Such at least one treatment element can be arranged anywhere on the base platform, for example in accordance with a specific treatment of a said person. In particular said at least one treatment element is arranged outside of the area of the at least one fill element. In particular said at least one treatment element is arranged inside an area where the foot of the person is located during treatment.

In an embodiment of the system according to the invention said at least one treatment element is optionally releasable connectable to the base platform, wherein said base platform and said at least one treatment element optionally comprise mutually connectable connecting elements.

An advantage of such an embodiment is that the at least one treatment element can be connected to the base platform at substantially any suitable position, for example in accordance with the specific treatment of a said person.

Said connecting means may be embodied in any suitable way. For example, but not limiting thereto, said treatment element may comprise at least one pin like element and the base platform may comprise at least one hole and preferably a plurality of holes divided over the upper surface, such that the treatment element may be connected to the base platform by inserting the pin like element into a said hole.

In an embodiment of the system according to the invention said at least one treatment element has a shape that is chosen from the group comprising a spherical shape, an optionally topped of pyramid, an optionally topped of cone, a cuboid shape, a bar shape, a cylindrical shape, or part thereof.

It will be clear for the skilled person that, although these shapes are suitable shapes for treating said person, these shapes are provided as an example only and that said treatment element may have any suitable shape.

In an embodiment of the system according to the invention said base platform comprises at least one optionally releasable top layer, which layer can be arranged on the upper surface or may define the upper surface, which layer is for example made of an elastically synthetic material.

Such top layer may be comfortable for the person standing on the base platform.

In an embodiment of the system according to the invention said measuring means comprise at least eight pressure sensors.

Said at least eight pressure sensors are preferably arranged at different, suitable locations for measuring the pressure at said different locations.

The applicant has found that eight pressure sensors may provide a sufficiently accurate pressure profile of each foot of the person, but if desired less or more pressure sensors can be provided.

In an embodiment of the system according to the invention said system comprises at least one of:
- an auditive stimulus, such as for example an environmental sound or music;
- a visual stimulus, such as for example an image or video, and
- a tactile stimulus, such as for example vibration means or thermal means.

Any one or more of such stimuli can be used for treating said person and applied as desired and/or in accordance with a specific treatment of the person and/or for relaxing said person.

In an embodiment of the system according to the invention said system comprises at least one muscle tension sensor for measuring a muscle tension of said person.

Using said at least one muscle tension sensor may provide insight into the state of the person and in particular if the person is relaxed or not.

In an embodiment of the system according to the invention said system comprises registration means for registering at least one of the following data:
- a or said measured pressure;
- a or said measured tension muscle;
- a or said at least one treatment element that is used and/or a position thereof, and
- a or said at least one stimulus that is used and/or a setting thereof.

Such registration means may be in particular arranged to register this information per individual person and preferably for a plurality of persons in an individual manner. An advantage of storing said information is for example that this information may be used in a next treatment.

Said system may further comprise a database for storing said data.

In an embodiment of the system according to the invention said system comprises a computing means which are arranged to determine based on said registered data which combination of treatment elements and/or stimuli and/or adjustable distance of the at least one fill element provide in a substantially equal pressure distribution of the or each foot and/or a low muscle tension.

An advantage thereof is that this can be used in the (next) treatment of said person, such that said person can be easily and/or quickly brought into his or her balanced position and/or relaxed state.

In an embodiment of the system according to the invention said system comprises control means for controlling said system.

Said control means may for example comprise said controller as described above.

In an embodiment of the system according to the invention said system comprises a scan device for scanning the position of the person.

Such a scan device may provide alternative or additional information on the position of the person.

The invention also relates to the use of a system as described above in any one or more of the above described embodiments and/or examples in any suitable combination, comprising the following steps:
a) providing a said system as described above in any one or more of the above described embodiments and/or examples in any suitable combination;
b) a said person standing on said base platform;
c) adjusting the adjustable distance of the at least one fill element.

Advantages of such a use and/or of the below embodiments of the use will be clear from the description relating to the system according to the invention.

In an embodiment of the use according the invention a system according to at least claim 2 is provided, and comprising step d) of measuring the pressure of a or each foot of said person, wherein step c) is performed by adjusting the adjustable distance of the at least one fill element in accordance with said measured pressure.

As described above with respect to the system according to the invention step c) may be performed by a person based on said displayed pressure or automatically by the system.

In an embodiment of the use according the invention, said use comprises the step of:
e) registering at least one of the following data:
- a or said measured pressure;
- a or said measured tension muscle;
- a or said at least one treatment element that is used and/or a position thereof, and
- a or said at least one stimulus that is used and/or a setting thereof.

As described above with respect to the system this method may be performed for multiple persons in an individualized manner.

In an embodiment of the use according the invention, said use comprises the step of:
f) determining based on said registered data which combination of treatment elements and/or stimuli and/or adjustable distance of the at least one fill element provide in a substantially equal pressure distribution of the or each foot and/or a low muscle tension.

This can be used in the (next) treatment of said person, in particular for example for adjusting the adjustable distance in step c) and/or in selecting and/or applying suitable treatment elements and/or stimuli and/or a respective position or setting thereof, such that said person can be easily and/or quickly brought into his or her balanced position and/or relaxed state.

Said use may further comprise any further or alternative step, for example in accordance with any of the embodiments of the system according to the invention. For example a step of using a at least one treatment element and/or at least one stimulus may be performed. For example a step of measuring a muscle tension may be performed.

The invention will be further elucidated with reference to figures, wherein:
Figures 1A - 1C show a system according to a first embodiment of the invention in a schematic perspective view, wherein figure 1A shows the system with a person standing on a base platform; figure 1B shows the system of figure 1A but without a cover plate, and figure 1C shows the principle of the displacement mechanism of a fill element in more detail;
Figures 2A and 2B schematically show the displacement of the fill element of figures 1A - 1C in more detail, respectively in a side view and rear view;
Figure 3 shows a system according to a second embodiment of the invention in a schematic perspective view;
Figure 4 shows the use of pressure sensors in a third embodiment of the system according to the invention in a schematical top view;
Figure 5 is a block diagram of a use of the system according to a first embodiment of the invention;
Figures 6A - 6C show a part of the system according to a fourth embodiment of the invention in various schematic perspective views, and
   Figure 7 shows exemplary fill elements that can be used in the system of figures 6A-6C. In the figures same features are referred to by same reference numerals, increased by one hundred (100) for further embodiments.

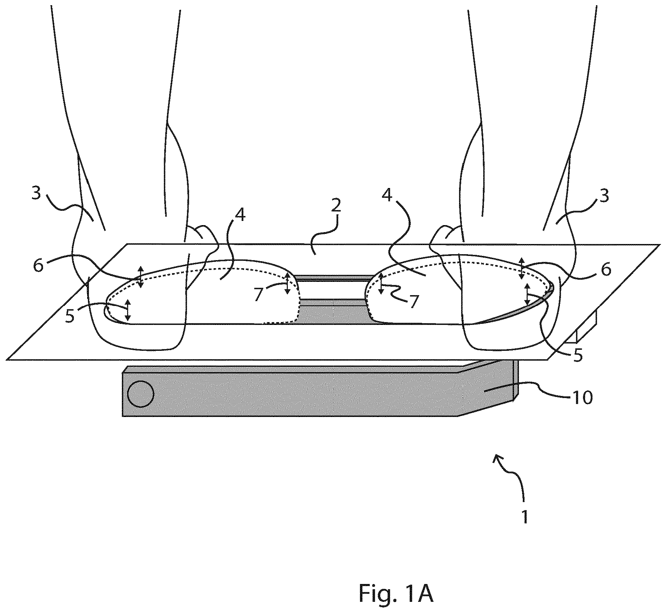

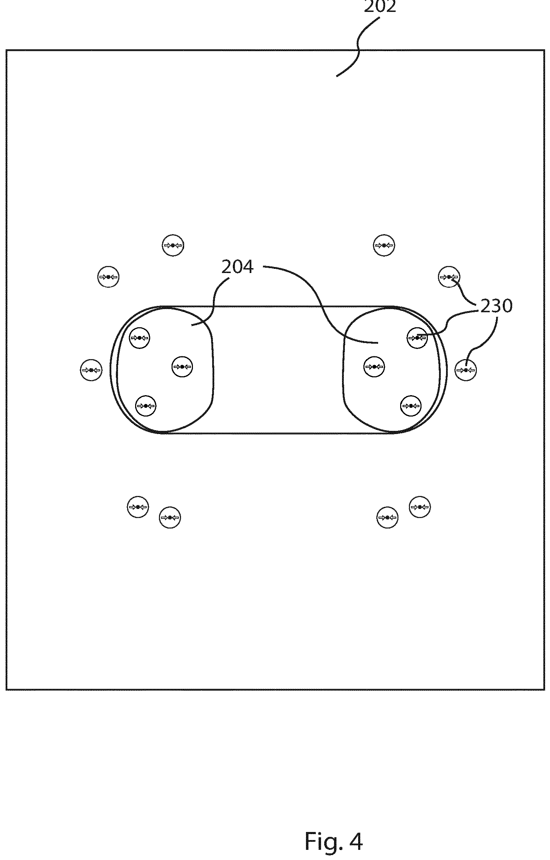

Figures 1A - 1C show a system for at least supporting a treatment of a person. Said system comprises a base platform 1 comprising in this embodiment a foot plate 2 as an upper surface on which a person can stand. The feet 3 of the person standing on the base platform 1 are shown. The foot plate 2 defines a substantially flat reference plane, except in the area of in this embodiment two fill elements 4. As is indicated by means of arrows 5, 6, 7 the two fill elements 4 are displaceable between a first position as shown by the dotted line fill elements 4 and a second position as shown by the unbroken line fill elements 4. In their second position an upper surface of the two fill elements 4 extends above said reference plane with an adjustable distance, such that the two fill elements each fill a respective space between said reference plane and a lower side of a respective foot 3 of said person. In this embodiment the two fill elements 4 also extend upwards with respect to the reference plane in their first position, but not at heigh as, i.e. with a smaller distance than, in the second position. The adjustable distance of the upper surface of the fill elements 4 in their second position can be chosen and in accordance therewith adjusted as desired. Preferably said distance is adjusted in accordance with a measured pressure, more in particular for example such that a pressure on both feet 3 is substantially equal and/or a pressure on a front part of the foot 3 is equal to a pressure on a rear part of the foot and/or a pressure on a left part of the foot 3 is equal to a pressure on a right part of the foot 3. An advantage of such any such equal pressure distribution is that said person may be able to stand on said base platform 1 in a balanced position. For the purpose of adjusting the adjustable distance in accordance with said measured pressure a plurality of pressure sensors could be provided, as is shown with respect to figure 4 and explained below.

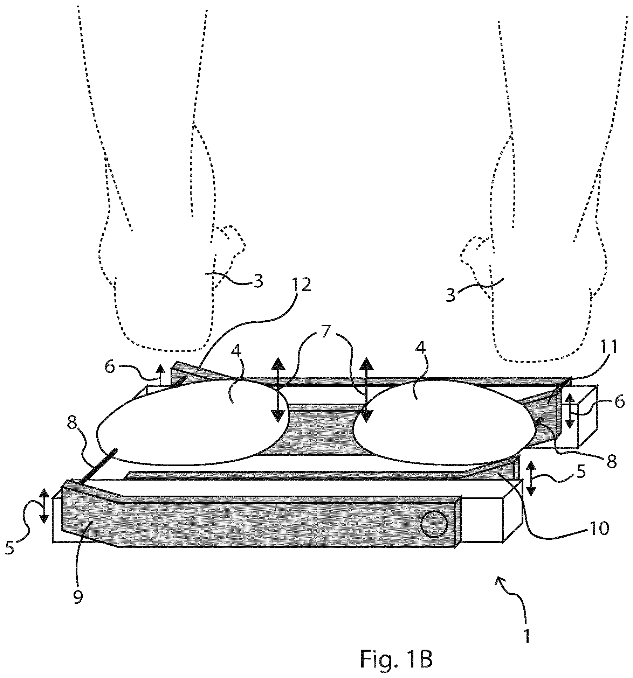

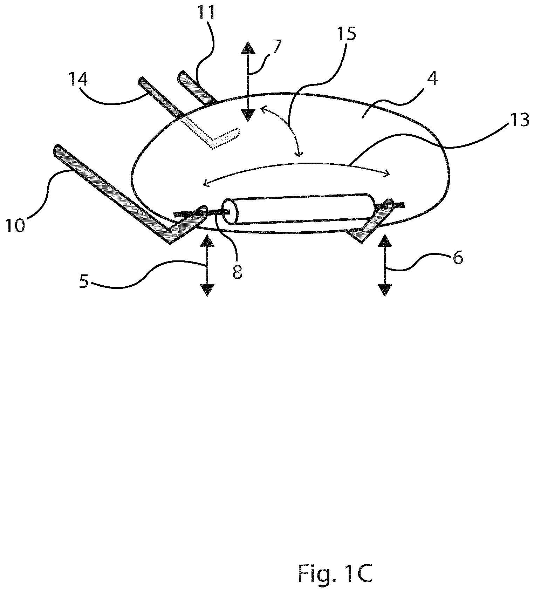

In this embodiment the two fill elements 4 are mechanically displaceable. As is shown in figures 1A - 1C each fill element 4 is attached to a respective carrier element 8, in this embodiment a substantially bar shaped carrier element 8. One carrier element 8 is attached to displaceable elements 9, 12 at the two opposing ends thereof, which displaceable elements 9, 12 form a first pair of displaceable elements 9, 12. The other carrier element 8 is attached to displaceable elements 10, 11 at the two opposing ends thereof, which displaceable elements 10, 11 form a second pair of displaceable elements 10, 11. The displaceable elements 9-12 are all individually moveable in a substantially vertical direction and both upwards and downwards, at least in the area where the carrier elements 8 are attached to the displaceable elements 9 - 12. As a result thereof, said fill elements 4 can be moved substantially in translation in said substantially vertical direction by moving any of said pairs of displaceable elements substantially simultaneously at substantially the same speed and over substantially the same distance in the same direction. By moving only one of the displaceable elements of a said pair and/or by moving the displaceable elements in opposite directions and/or at different speeds, said fill elements 4 can be moved in rotation or a combination of rotation and translation. Figure 1C shows the principle of this movement, wherein the displacement of the carrier element 8 by means of displacement elements 10, 11 is shown by means of arrows 5, 6, and from which it is clear that the carrier element 8 is moved in translation in the vertical direction if the displacement elements 10, 11 move in the same direction at substantially the same speed and/or is (also) rotated along arrow 13, i.e. tilted, if the displacement elements 10, 11 move at different speeds and/or in opposite directions. Not visible in figures 1A and 1B but as shown in figure 1C, a further displacement element 14 can be provided for each fill element 4 individually that is able to displace said fill element 4 in substantially the vertical direction as indicated by arrow 7. As a result thereof each fill element 4 is rotatable substantially along a rotation axis defined by carrier element 8 in the direction of arrow 15.

It will be clear for the skilled person that the mechanical embodiment of figures 1A - 1C is only exemplary and not limiting. It will be clear that any alternative mechanical mechanism for displacing the at least one fill element may be provided, wherein preferably any fill element provided may be displaceable in an individual manner and/or in translation and/or rotation.

As is further shown in figures 1A - 1C the two fill elements 4 each have a shape that is substantially adapted to a said foot 3 of said person. As a result of said shape the space between the foot arch and the foot plate 2 can be substantially completely filled. As a results of the shape the distance of the upper surface of the fill element 4 with respect to the reference plane is different, i.e. variable, per surface area zone of the fill element 4. It is noted that the fill elements 4 could be individually provided fill elements that are made in accordance with the shape of a specific foot. Such fill elements could be releasably connected to the base platform 1 and therefore be replaceable if a different person is to be treated. Alternatively it can be a general shape that is adapted to a general shape of the foot.

A motor, for example an electromotor, may be provided for displacing the displacement elements 9 - 12. A controller may be used for controlling the movement of the displacement elements 9 - 12. Moving the displacement elements 9-12 and thereby the adjustment of the adjustable distance of the fill elements 4 may be performed manually or automatically.

If desired at least one stimulus may be provided in the treatment of said person. Such a stimulus may for example be:
- an auditive stimulus, such as for example an environmental sound or music;
- a visual stimulus, such as for example an image or video, and
- a tactile stimulus, such as for example vibration means or thermal means.

If desired said system may comprise at least one muscle tension sensor for measuring a muscle tension of said person. Such a sensor may be used for measuring the muscle tension and thereby if the person is relaxed or not

Said system may comprise a registration means for registering at least one of the following data:
- a or said measured pressure;
- a or said measured muscle tension;
- a or said at least one treatment element that is used, and
- a or said at least one stimulus that is used and/or a setting thereof.

The system may comprise a computing means which is arranged to determine based on said registered data which combination of treatment elements and/or stimuli and/or adjustable distance of the at least one fill element provide in a substantially equal pressure distribution of the or each foot and/or a low muscle tension.

The system may comprise a scan device for scanning the position of the person.

The above described features are not shown in the figures but are known per se to the skilled person. The skilled person would thus know how to apply these features in the system according to the invention based on the description of these features.

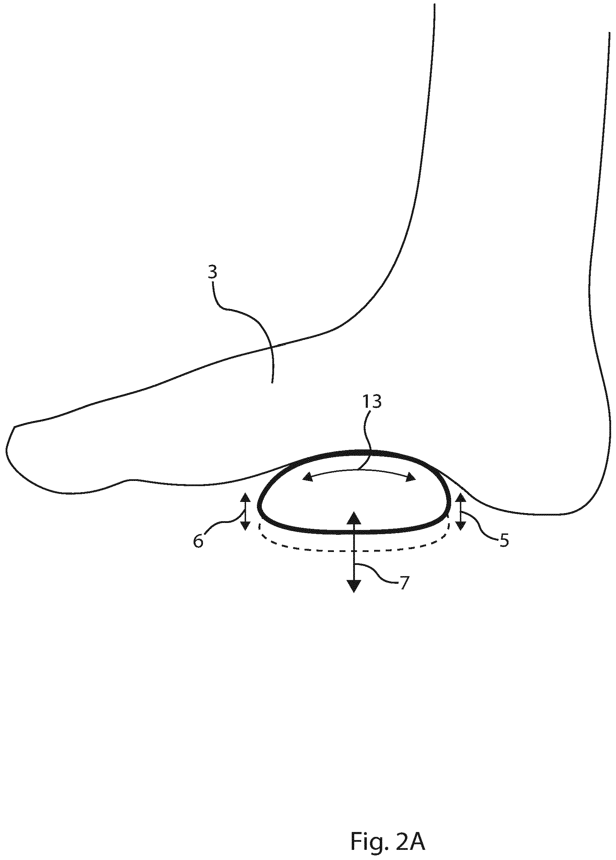

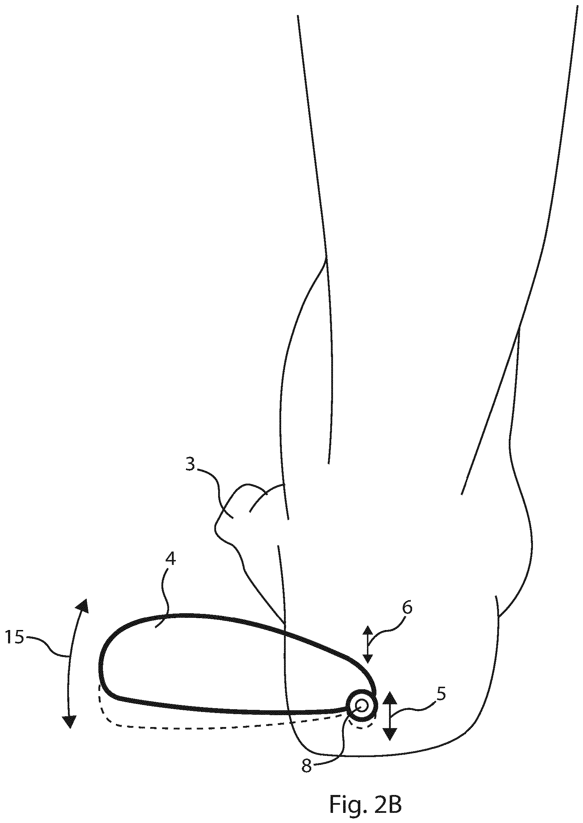

Figures 2A and 2B show the principle of displacement of a said fill element 4 of the first embodiment with respect to the foot 3, wherein fig. 2A shows a side view of the foot 3 and figure 2B shows a rear view of the foot 3. For a further description of the movement of the fill element 4 the reader is referred to the description of figures 1A - 1C and in particular fig. 1C.

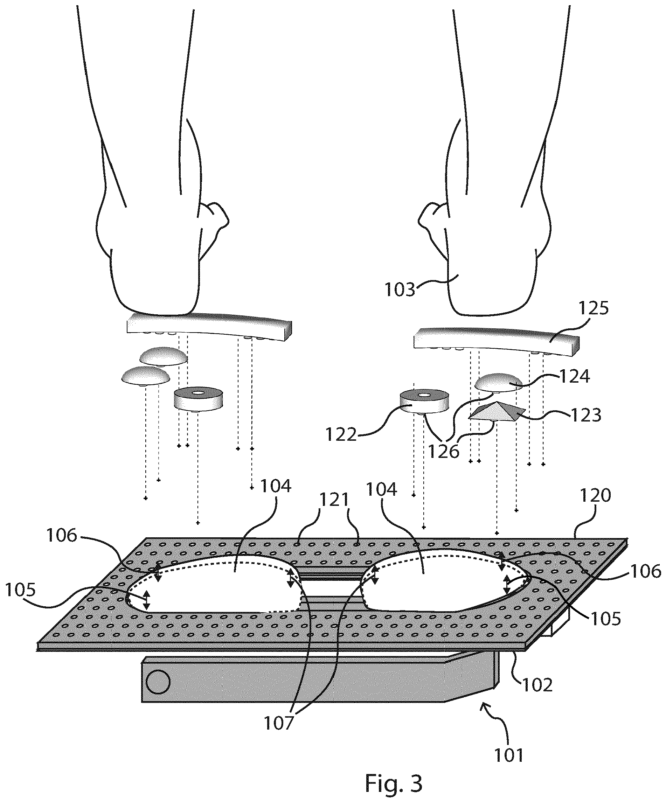

Figure 3 shows a second embodiment of the system. Only the differences with the first embodiment of figures 1A - 1C, 2A, 2B, will be described here. Fur a further description of the system according to the second embodiment the reader is referred to the description of figures 1A-1C and 2A, 2B.

The system of figure 3 differs from the first embodiment in that an optionally releasable top layer 120 is arranged on the foot plate 102. Said top layer 120 comprises a plurality of holes 121. In this embodiment treatment elements 122 - 125 are provided which can be connected to the top layer 120 by inserting a pin 126 thereof in a hole 121. This way the treatment elements 122 - 125 can be connected to the top layer 120 at any suitable location. In their connected state in which the treatment elements 122 - 125 are connected to the top layer 120 they extend upward out of said reference plane. In figur3 four different treatment elements 122 - 125 are shown. Treatment element 122 is substantially disk shaped, i.e. the shape of a cylinder. Treatment element 123 is substantially pyramid shaped. Treatment element 124 is substantially shaped as half a sphere. Treatment element 125 has substantially the shape of a bent beam or bar.

It will be clear for the skilled person that the treatment elements may have any suitable shape and that any suitable number of treatment elements may be used. It will further be clear that the number of pins 126 that a treatment element comprises may for example be one for a relatively small treatment element and/or for rotationally symmetrical treatment elements, and may be more than one, for example two, for larger treatment elements and/or for treatment elements that are not rotationally symmetric. It will further be clear that the treatment elements may be connected or attached to the top layer 120, foot plate 102, or any other upper surface of the base platform 101 in any suitable way and this is in no way limited to the shown pin and hole connection.

The top layer 120 of the second embodiment may be embodied in any suitable way and/or made of any suitable material. For example said top layer 120 may be made of an elastically synthetic material, which material may be a convenient and/or relatively soft material for the person to stand on.

Figure 4 shows a third embodiment of the system. Only the differences with the first embodiment of figures 1A - 1C, 2A, 2B, will be described here. Fur a further description of the system according to the second embodiment the reader is referred to the description of figures 1A-1C and 2A, 2B.

Figure 4 shows that the upper surface 202 and the upper surface of the two fill elements 204 may comprise a plurality of pressure sensors 230. In this exemplary embodiment sixteen pressure sensors 230 are shown, equally divided per foot, so eight per foot. The pressure sensors 230 are arranged for measuring the pressure of each foot such that the displacement of the fill elements 204 may be in accordance with the measured pressure, as described above. The measured pressure may be displayed such that for example a practitioner may adjust the adjustable distance of the fill elements 204 in accordance with the displayed pressure and/or may be used by a said controller for controlling the adjustment of the adjustable distance. The controller may be operatively connected to the pressure sensors.

Said pressure sensors may for example alternatively arranged in a said top layer.

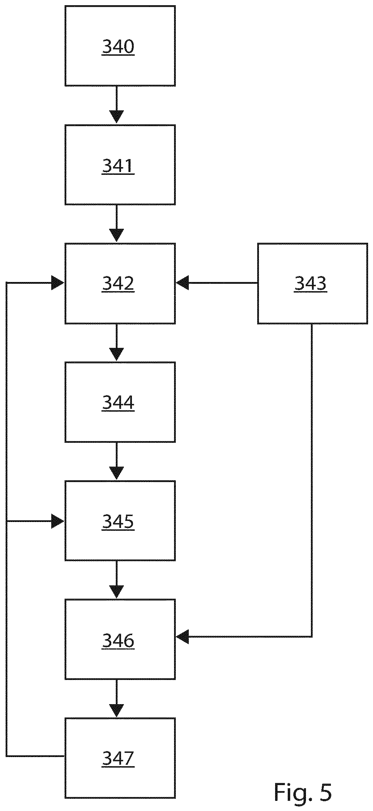

Figure 5 shows a block diagram of an exemplary use of said system as described in any of the three embodiments of figures 1-4. This figure shows that the use of this embodiment comprises step 340 of providing a system, for example according to any of the three embodiments of figures 1-4. In step 341 a person will stand on the base platform, in particular on the upper surface thereof. In step 342 the adjustable distance of the at least one fill element is adjusted.

In an optional step 343 the pressure of each foot of said person is measured. If such a step 343 is provided step 342 may be performed by adjusting the adjustable distance of the at least one fill element in accordance with said measured pressure.

In an optional step 344 at least one treatment element is used by connecting or attaching that treatment element to the upper surface of the base platform and/or at least one stimulus is used.

In an optional step 345 the muscle tension of the person is measured.

In an optional step 346 at least one of the following data is registered:
- said measured pressure;
- said measured tension muscle;
- said at least one treatment element that is used, and
- said at least one stimulus that is used and/or a setting thereof.

In an optional step 347 it is determined based on data registered in step 346 which combination of treatment elements and/or stimuli and/or adjustable distance of the at least one fill element provide in a substantially equal pressure distribution of the or each foot and/or a low muscle tension. This may for example be used in a next treatment of the person and more in particular for example for adjusting the adjustable distance of the at least one fill element in step 342 and/or in step 344 for choosing suitable stimuli and/or treatment elements and/or a suitable setting and/or position thereof.

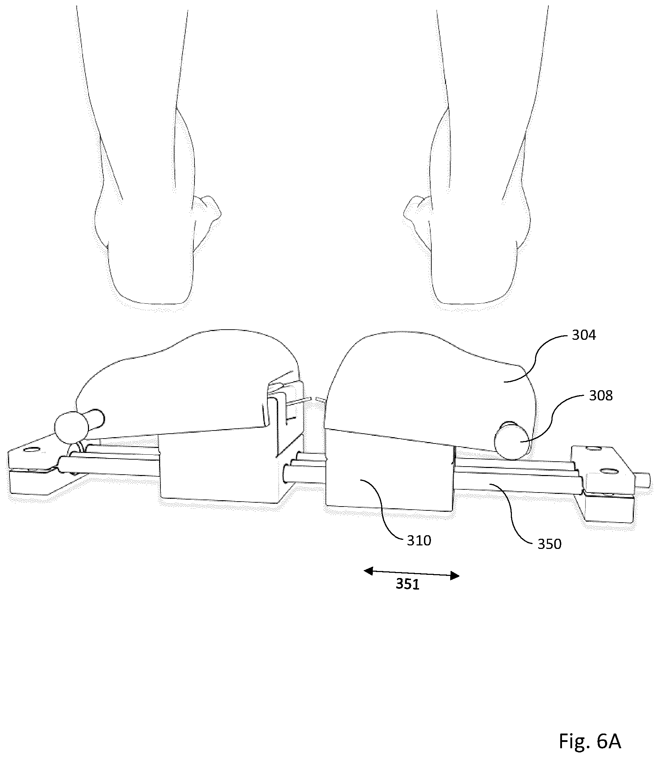

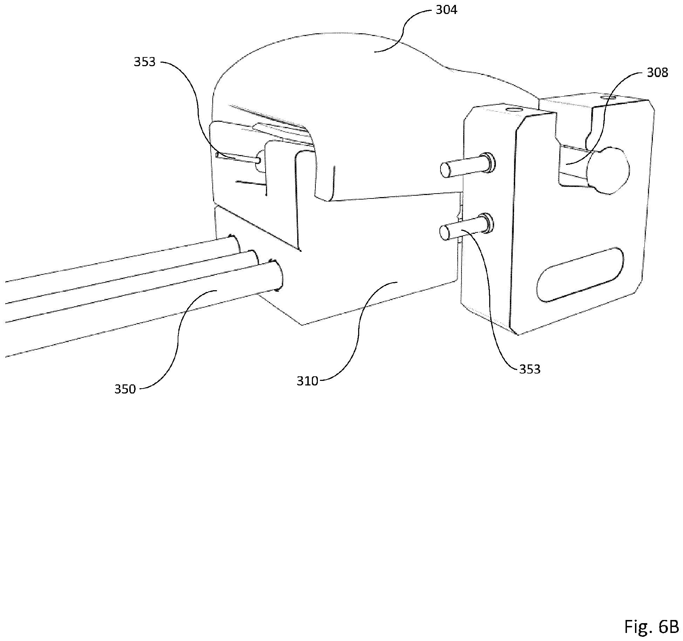

Figures 6A - 6C show a fourth embodiment of the displacement mechanism and fill elements of the system according to the invention. Only the differences with the first embodiment of figures 1A - 1C, 2A, 2B, will be described here. For a further description of the system according to the fourth embodiment the reader is referred to the description of figures 1A - 1C and 2A, 2B.

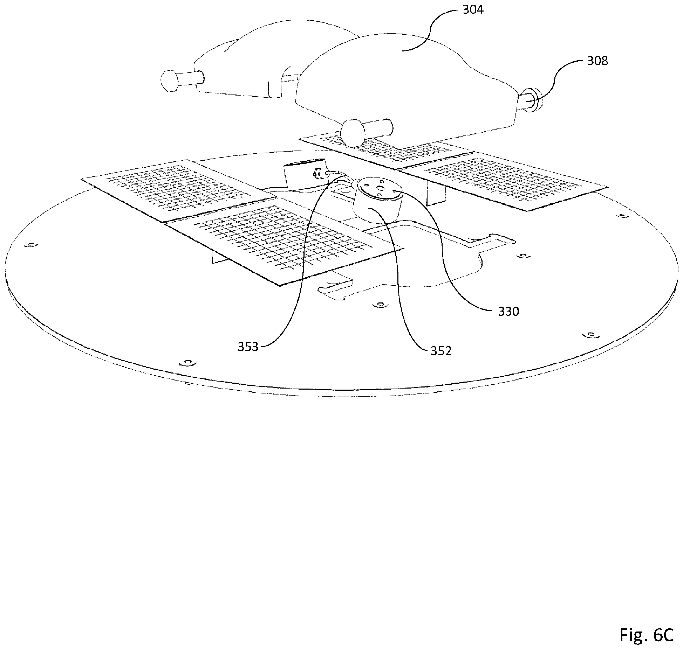

The system of figures 6A - 6C differs from the first embodiment in that the fill elements 304 are freely rotatable about a rotation axis defined by a carrier element or rod 308. Displacement elements 310 for displacing a respective fill element 304 are moveable along guiding rods 350. When the displacement elements 310 move away from their respective rod 308 the fill element 304 is rotated upwards and when the displacement elements 310 move towards their respective rod 308 the fill element 304 is rotated upwards. This direction of movement of the displacement elements 310 is shown by arrow 351. Figure 6C further shows that the fill element 304 and rod 308 are provided together as a replaceable element. This allows different shapes of fill elements 304 to be used, for example in dependence with the shape of a foot arch of the user of the system. Figure 6C further shows that the pressure sensors 330 are in this fourth embodiment provided on respective measurement heads 352, which are covered by respective fill elements 304. Electrical connections 353 are shown which allow for electrical powering the movement of the displacement elements 310 and measurement heads 352.

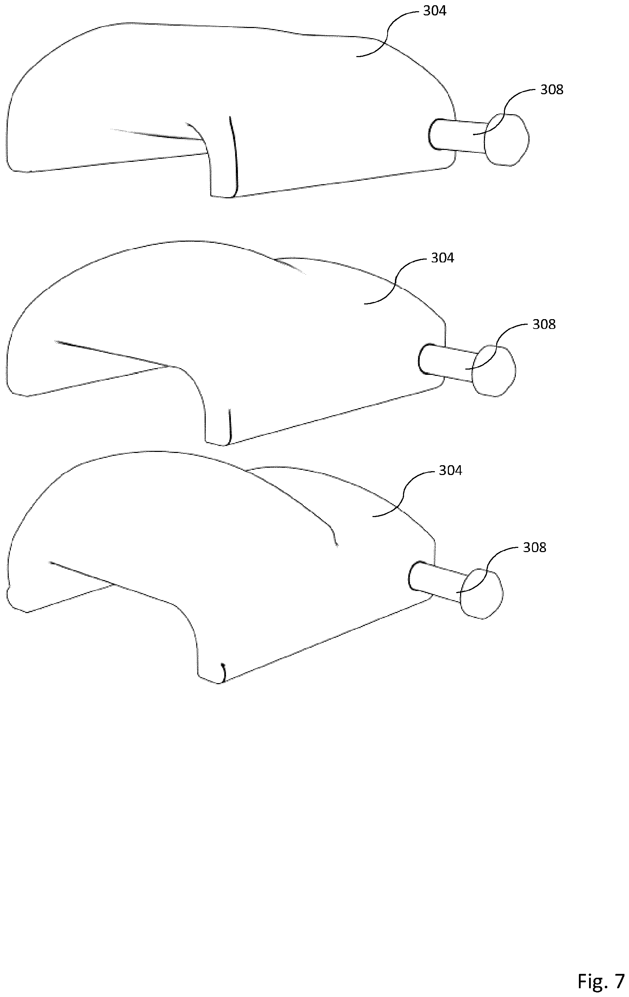

Figure 7 shows various examples of fill elements 304 that can be used in the system of figures 6A - 6C.

It will be clear for the skilled person that the method steps may be performed in any suitable order, optionally different from the order as shown. It will further be clear that said method may comprise less or more steps, to be performed at any suitable stage, for example after or between the shown steps.

It is noted that the invention is not limited to the shown embodiments but also extends to variants within the scope of the appended claims.

## Claims

1. System for at least supporting a treatment of a person, comprising a base platform (1) for receiving a foot or feet of said person, wherein said base platform comprises at least one upper surface which defines a substantially flat reference plane, and at least one fill element (4), **characterized in that** said at least one fill element is displaceable between a first position and a second position, in which second position an upper surface of said at least one fill element extends above said reference plane with an adjustable distance, such that said at least one fill element fills a space between said reference plane and a lower side of a or each foot of said person.

2. System according to claim 1, wherein said system comprises measuring means for measuring a pressure of the or each foot of the person, wherein said system is arranged to adjust the adjustable distance of said at least one fill element based on the measured pressure and/or comprises displaying means for displaying the measured pressure, optionally wherein said means comprise at least eight pressure sensors.

3. System according to claim 1 or 2, wherein said adjustable distance is adjusted such that:
- a pressure on both feet is substantially equal; and/or
- a pressure on a front part of the foot is equal to a pressure on a rear part of the foot; and/or
- a pressure on a left part of the foot is equal to a pressure on a right part of the foot.

4. System according to any of the preceding claims, comprising a driving means for displacing the at least one fill element between its first and second position, wherein said driving means are arranged for displacing the at least one fill element mechanically and/or by means of a gas and/or by means of a liquid.

5. System according to any of the preceding claims, wherein the upper surface of the at least one fill element defines a number of surface area zones, wherein the adjustable distance is variable and/or individually adjustable per surface area zone.

6. System according to any of the preceding claims, wherein said at least one fill element has a shape that is substantially adapted to the or each foot of said person.

7. System according to any of the preceding claims, wherein said displacement takes place using rotation and/or translation of the at least one fill element.

8. System according to any of the preceding claims, wherein said at least one fill element is arranged and/or a distance between at least two fill elements is chosen such that a straight line is defined between an outer side of a hip bone and a second toe of the person when said person stands on said base platform and said at least one or two fill element(s) fill(s) said space.

9. System according to any of the preceding claims, wherein said system comprises at least one treatment element that extends out of said reference plane, wherein optionally said at least one treatment element is optionally releasably connectable to the base platform, wherein said base platform and said at least one treatment element optionally comprise mutually cooperating connecting elements, wherein optionally said at least one treatment element has a shape that is chosen from the group comprising a spherical shape, an optionally topped of pyramid, an optionally topped of cone, a cuboid shape, a bar shape, a cylindrical shape, or part thereof.

10. System according to any of the preceding claims, wherein said base platform comprises at least one optionally releasable top layer, which layer can be arranged on the upper surface or may define the upper surface, which layer is for example made of an elastically synthetic material.

11. System according to any of the preceding claims, comprising at least one of:
- an auditive stimulus, such as for example an environmental sound or music;
- a visual stimulus, such as for example an image or video;
- a tactile stimulus, such as for example vibration means or thermal means, and
- at least one muscle tension sensor for measuring a muscle tension of said person.

12. System according to any of the preceding claims, wherein said system comprises registration means for registering at least one of the following data:
- a or said measured pressure;
- a or said measured tension muscle;
- a or said at least one treatment element that is used and/or a position thereof, and
- a or said at least one stimulus that is used and/or a setting thereof.

13. System according to any of the preceding claims, wherein said system comprises at least one of:
- a computing means which is arranged to determine based on said registered data which combination of treatment elements and/or stimuli and/or adjustable distance of the at least one fill element provide in a substantially equal pressure distribution of the or each foot and/or a low muscle tension;
- a control means for controlling said system, and
- a scan device for scanning the position of the person.

14. Use of a system according to any of the preceding claims, comprising the following steps:
a) providing a said system according to any of claims 1- 13;
b) a said person standing on said base platform, and
c) adjusting the adjustable distance of the at least one fill element.

15. Use of a system according to claim 14, comprising at least one of the following steps:
d) measuring the pressure of a or each foot of said person, wherein step c) is performed by adjusting the adjustable distance of the at least one fill element in accordance with said measured pressure, wherein a system according to at least claim 2 is provided;
e) registering at least one of the following data:
- a or said measured pressure;
- a or said measured tension muscle;
- a or said at least one treatment element that is used and/or a position thereof, and
- a or said at least one stimulus that is used and/or a setting thereof, and
f) determining based on said registered data which combination of treatment elements and/or stimuli and/or adjustable distance of the at least one fill element provide in a substantially equal pressure distribution of the or each foot and/or a low muscle tension.

## Patentansprüche

1. System zum mindestens Unterstützen einer Behandlung einer Person, umfassend eine Basisplattform (1) zum Aufnehmen eines Fußes oder mehrerer Füße der Person, wobei die Basisplattform mindestens eine obere Oberfläche umfasst, die eine im Wesentlichen flache Referenzebene definiert, und mindestens ein Füllelement (4), **dadurch gekennzeichnet, dass** das mindestens eine Füllelement ist zwischen einer ersten Position und einer zweiten Position verschiebbar, wobei in der zweiten Position eine obere Oberfläche des mindestens einen Füllelements sich über der Referenzebene mit einem einstellbaren Abstand erstreckt, sodass das mindestens eine Füllelement einen Raum zwischen der Referenzebene und einer unteren Seite eines oder jedes Fußes der Person ausfüllt.

2. System nach Anspruch 1, wobei das System Messmittel zum Messen eines Drucks des oder jedes Fußes der Person umfasst, wobei das System angeordnet ist, um den einstellbaren Abstand des mindestens einen Füllelements basierend auf dem gemessenen Druck einzustellen und/oder Anzeigemittel zum Anzeigen des gemessenen Drucks umfasst, optional wobei die Mittel mindestens acht Drucksensoren umfassen.

3. System nach Anspruch 1 oder 2, wobei der einstellbare Abstand derart eingestellt wird, dass:
- ein Druck auf beide Füße ist im Wesentlichen gleich; und/oder
- ein Druck auf einen vorderen Teil des Fußes gleich einem Druck auf einen hinteren Teil des Fußes ist; und/oder
- ein Druck auf den linken Teil des Fußes gleich einem Druck auf den rechten Teil des Fußes ist.

4. System nach einem der vorstehenden Ansprüche, umfassend ein Antriebsmittel zum Verschieben des mindestens einen Füllelements zwischen seiner ersten und zweiten Position, wobei die Antriebsmittel zum mechanischen Verschieben des mindestens einen Füllelements und/oder mittels eines Gases und/oder mittels einer Flüssigkeit angeordnet sind.

5. System nach einem der vorstehenden Ansprüche, wobei die obere Oberfläche des mindestens einen Füllelements eine Anzahl von Oberflächenzonen definiert, wobei der einstellbare Abstand variabel und/oder pro Oberflächenzone individuell einstellbar ist.

6. System nach einem der vorstehenden Ansprüche, wobei das mindestens eine Füllelement eine Form aufweist, die im Wesentlichen an den oder jeden Fuß der Person angepasst ist.

7. System nach einem der vorstehenden Ansprüche, wobei die Verschiebung unter Verwendung von Drehung und/oder Translation des mindestens einen Füllelements stattfindet.

8. System nach einem der vorstehenden Ansprüche, wobei das mindestens eine Füllelement angeordnet ist, und/oder ein Abstand zwischen mindestens zwei Füllelementen gewählt ist, derart, dass eine gerade Linie zwischen einer Außenseite eines Hüftknochens und einem zweiten Zeh der Person definiert ist, wenn die Person auf der Basisplattform steht und das mindestens eine oder die zwei Füllelemente den Raum ausfüllen.

9. System nach einem der vorstehenden Ansprüche, wobei das System mindestens ein Behandlungselement umfasst, das sich aus der Referenzebene heraus erstreckt, wobei optional das mindestens eine Behandlungselement mit der Basisplattform optional lösbar verbindbar ist, wobei die Basisplattform und das mindestens eine Behandlungselement miteinander zusammenwirkende Verbindungselemente optional umfassen, wobei optional das mindestens eine Behandlungselement eine Form aufweist, die aus der Gruppe ausgewählt ist, umfassend eine Kugelform, eine optional gekappte Pyramide, einen optional gekappten Kegel, eine Quaderform, eine Balkenform, eine Zylinderform oder einen Teil davon umfasst.

10. System nach einem der vorstehenden Ansprüche, wobei die Basisplattform mindestens eine optional lösbare Oberschicht umfasst, wobei die Schicht auf der oberen Oberfläche angeordnet sein kann oder die obere Oberfläche definieren kann, wobei die Schicht zum Beispiel aus einem elastischen Kunststoffmaterial gefertigt ist.

11. System nach einem der vorstehenden Ansprüche, umfassend mindestens eines von:
- einem akustischen Reiz, wie zum Beispiel einem Umgebungsgeräusch oder Musik;
- einem visuellen Reiz, wie zum Beispiel einem Bild oder einem Video;
- einem taktilen Reiz, wie zum Beispiel Vibrations- oder Wärmemitteln, und
- mindestens einem Muskelspannungssensor zum Messen einer Muskelspannung der Person.

12. System nach einem der vorstehenden Ansprüche, wobei das System Registrierungsmittel zum Registrieren von mindestens einem aus den folgenden Daten umfasst:
- einem oder dem gemessenen Druck;
- einem oder dem gemessenen Spannungsmuskel;
- einem oder dem mindestens einen Behandlungselement, das verwendet wird und/oder einer Position davon und
- einem oder dem mindestens einen Reiz, der verwendet wird und/oder einer Einstellung davon.

13. System nach einem der vorstehenden Ansprüche, wobei das System mindestens eines umfasst von:
- einem Rechenmittel, das angeordnet ist, um basierend auf den registrierten Daten zu bestimmen, welche Kombination von Behandlungselementen und/oder Reizen und/oder einstellbarem Abstand des mindestens einen Füllelements eine im Wesentlichen gleichmäßige Druckverteilung des oder jedes Fußes und/oder eine geringe Muskelspannung bereitstellt;
- einem Steuermittel zum Steuern des Systems und
- einer Scan-Vorrichtung zum Abtasten der Position der Person.

14. Verwendung eines Systems nach einem der vorstehenden Ansprüche, umfassend die folgenden Schritte:
a) Bereitstellen eines solchen Systems nach einem der Ansprüche 1 bis 13;
b) eine Person, die auf der Basisplattform steht, und
c) Einstellen des einstellbaren Abstands des mindestens einen Füllelements.

15. Verwendung eines Systems nach Anspruch 14, umfassend mindestens einen der folgenden Schritte:
d) Messen des Drucks eines oder jedes Fußes der Person, wobei Schritt c) durch Einstellen des einstellbaren Abstands des mindestens einen Füllelements gemäß dem gemessenen Druck durchgeführt wird, wobei ein System nach mindestens Anspruch 2 bereitgestellt wird;
e) Registrieren mindestens eines aus den folgenden Daten:
- einem oder dem gemessenen Druck;
- einem oder dem gemessenen Spannungsmuskel;
- einem oder dem mindestens einen Behandlungselement, das verwendet wird und/oder einer Position davon und
- einem oder dem mindestens einen Reiz, der verwendet wird und/oder einer Einstellung davon, und
f) Bestimmen basierend auf den registrierten Daten, welche Kombination von Behandlungselementen und/oder Reizen und/oder einstellbarem Abstand des mindestens einen Füllelements eine im Wesentlichen gleichmäßige Druckverteilung des oder jedes Fußes und/oder eine geringe Muskelspannung bereitstellt.

## Revendications

1. Système permettant au moins de soutenir un traitement d'une personne, comprenant une plate-forme de base (1) pour recevoir un pied ou des pieds de ladite personne, dans lequel ladite plate-forme de base comprend au moins une surface supérieure qui définit un plan de référence sensiblement plat, et au moins un élément de remplissage (4), **caractérisé en ce que** ledit au moins un élément de remplissage est déplaçable entre une première position et une seconde position, dans laquelle seconde position, une surface supérieure dudit au moins un élément de remplissage s'étend au-dessus dudit plan de référence avec une distance ajustable, de telle sorte que ledit au moins un élément de remplissage remplit un espace entre ledit plan de référence et un côté inférieur d'un ou de chaque pied de ladite personne.

2. Système selon la revendication 1, dans lequel ledit système comprend des moyens de mesure pour mesurer une pression du ou de chaque pied de la personne, dans lequel ledit système est agencé pour ajuster la distance ajustable dudit au moins un élément de remplissage en fonction de la pression mesurée et/ou comprend des moyens d'affichage pour afficher la pression mesurée, éventuellement dans lequel lesdits moyens comprennent au moins huit capteurs de pression.

3. Système selon la revendication 1 ou 2, dans lequel ladite distance ajustable est ajustée de telle sorte que :
- une pression sur les deux pieds est sensiblement égale ; et/ou
- une pression sur une partie avant du pied est égale à une pression sur une partie arrière du pied ; et/ou
- une pression sur une partie gauche du pied est égale à une pression sur une partie droite du pied.

4. Système selon l'une quelconque des revendications précédentes, comprenant des moyens d'entraînement pour déplacer l'au moins un élément de remplissage entre sa première et sa seconde position, dans lequel lesdits moyens d'entraînement sont agencés pour déplacer l'au moins un élément de remplissage mécaniquement et/ou au moyen d'un gaz et/ou au moyen d'un liquide.

5. Système selon l'une quelconque des revendications précédentes, dans lequel la surface supérieure de l'au moins un élément de remplissage définit un certain nombre de zones de surface, dans lequel la distance ajustable est variable et/ou ajustable individuellement par zone de surface.

6. Système selon l'une quelconque des revendications précédentes, dans lequel ledit au moins un élément de remplissage a une forme qui est sensiblement adaptée au pied ou à chaque pied de ladite personne.

7. Système selon l'une quelconque des revendications précédentes, dans lequel ledit déplacement s'effectue à l'aide de la rotation et/ou de la translation de l'au moins un élément de remplissage.

8. Système selon l'une quelconque des revendications précédentes, dans lequel ledit au moins un élément de remplissage est agencé et/ou une distance entre au moins deux éléments de remplissage est choisie de telle sorte qu'une ligne droite est définie entre un côté extérieur d'un os de la hanche et un deuxième orteil de la personne lorsque ladite personne se tient debout sur ladite plate-forme de base et que ledit au moins un ou deux élément(s) de remplissage remplit(remplissent) ledit espace.

9. Système selon l'une quelconque des revendications précédentes, dans lequel ledit système comprend au moins un élément de traitement qui s'étend hors dudit plan de référence, dans lequel, éventuellement, ledit au moins un élément de traitement peut éventuellement être relié de manière amovible à la plate-forme de base, dans lequel ladite plate-forme de base et ledit au moins un élément de traitement comprennent éventuellement des éléments de liaison coopérant mutuellement, dans lequel éventuellement ledit au moins un élément de traitement a une forme qui est choisie dans le groupe comprenant une forme sphérique, une pyramide éventuellement coiffée, un cône éventuellement coiffé, une forme cubique, une forme de barre, une forme cylindrique, ou une partie de celles-ci.

10. Système selon l'une quelconque des revendications précédentes, dans lequel ladite plate-forme de base comprend au moins une couche supérieure éventuellement amovible, laquelle couche peut être agencée sur la surface supérieure ou peut définir la surface supérieure, laquelle couche est par exemple constituée d'un matériau élastiquement synthétique.

11. Système selon l'une quelconque des revendications précédentes, comprenant au moins l'un parmi :
- un stimulus auditif, tel que par exemple un son environnemental ou de la musique ;
- un stimulus visuel, tel que par exemple une image ou une vidéo ;
- un stimulus tactile, tel que par exemple des moyens vibratoires ou des moyens thermiques, et
- au moins un capteur de tension musculaire pour mesurer une tension musculaire de ladite personne.

12. Système selon l'une quelconque des revendications précédentes, dans lequel ledit système comprend des moyens d'enregistrement pour enregistrer au moins l'une des données suivantes :
- une ou ladite pression mesurée ;
- une ou ladite tension musculaire mesurée ;
- un ou ledit au moins un élément de traitement qui est utilisé et/ou une position de celui-ci, et
- un ou ledit au moins un stimulus qui est utilisé et/ou un réglage de celui-ci.

13. Système selon l'une quelconque des revendications précédentes, dans lequel ledit système comprend au moins l'un parmi :
- un moyen informatique qui est agencé pour déterminer, en fonction desdites données enregistrées, quelle combinaison d'éléments de traitement et/ou de stimuli et/ou de distance ajustable de l'au moins un élément de remplissage fournit une répartition sensiblement égale de la pression sur le ou chaque pied et/ou une faible tension musculaire ;
- un moyen de commande pour commander ledit système, et
- un dispositif de balayage pour balayer la position de la personne.

14. Utilisation d'un système selon l'une quelconque des revendications précédentes, comprenant les étapes suivantes :
a) la fourniture d'un dit système selon l'une quelconque des revendications 1 à 13 ;
b) une dite personne se tenant sur ladite plate-forme de base, et
c) l'ajustement de la distance ajustable de l'au moins un élément de remplissage.

15. Utilisation d'un système selon la revendication 14, comprenant au moins l'une des étapes suivantes :
d) la mesure de la pression d'un ou de chaque pied de ladite personne, dans laquelle l'étape c) est réalisée en ajustant la distance ajustable de l'au moins un élément de remplissage conformément à ladite pression mesurée, dans laquelle un système selon au moins la revendication 2 est fourni ;
e) l'enregistrement d'au moins l'une des données suivantes :
- une ou ladite pression mesurée ;
- une ou ladite tension musculaire mesurée ;
- un ou ledit au moins un élément de traitement qui est utilisé et/ou une position de celui-ci, et
- un ou ledit au moins un stimulus qui est utilisé et/ou un réglage de celui-ci, et
f) la détermination, en fonction desdites données enregistrées, de quelle combinaison d'éléments de traitement et/ou de stimuli et/ou de distance ajustable de l'au moins un élément de remplissage fournit une répartition sensiblement égale de la pression sur le ou chaque pied et/ou une faible tension musculaire.
